Europäisches Patentamt

European Patent Office

Office européen des brevets

(19)

(11) Numéro de publication: **0 511 049 B1**

(12) **FASCICULE DE BREVET EUROPEEN**

(45) Date de publication de fascicule du brevet: **05.10.94**   (51) Int. Cl.⁵: **A61G 13/04**, A61B 6/04

(21) Numéro de dépôt: **92401029.1**

(22) Date de dépôt: **13.04.92**

(54) **Chassis de table d'examen medical.**

(30) Priorité: **18.04.91 FR 9104815**

(43) Date de publication de la demande:
**28.10.92 Bulletin 92/44**

(45) Mention de la délivrance du brevet:
**05.10.94 Bulletin 94/40**

(84) Etats contractants désignés:
**BE DE ES IT NL**

(56) Documents cités:
**EP-A- 0 119 910**
**EP-A- 0 389 332**
**FR-A- 1 357 371**
**US-A- 2 680 046**

(73) Titulaire: **GENERAL ELECTRIC CGR S.A.**
**100, rue Camille-Desmoulins**
**F-92130 Issy les Moulineaux (FR)**

(72) Inventeur: **Langenaecken, René**
**Cabinet Ballot-Schmit,**
**7, rue Le Sueur**
**F-75116 Paris (FR)**
Inventeur: **Martin, Claude**
**Cabinet Ballot-Schmit,**
**7, rue Le Sueur**
**F-75116 Paris (FR)**

(74) Mandataire: **Schmit, Christian Norbert Marie**
**et al**
**Cabinet Ballot-Schmit**
**7, rue Le Sueur**
**F-75116 Paris (FR)**

Rank Xerox (UK) Business Services
(3. 10 / 3.0 9/3.3.3)

## Description

La présente invention a pour objet une table d'examen médical utilisable essentiellement dans le domaine de la radiologie. On pourrait cependant imaginer qu'elle puisse être utilisée dans d'autres domaines de la médecine par exemple en médecine nucléaire ou dans le domaine des ultrasons. La particularité des tables d'examen médical de radiologie est qu'elles doivent permettre d'orienter le patient dans l'espace de diverses manières, tout en permettant par ailleurs des fonctionnalités diverses, telles que la myélographie, la tomographie, la cardiographie, l'angiographie etc. ... .

Une table d'examen médical comporte normalement un socle posé ou fixé sur le sol et un châssis qui porte un panneau porte-patient. Le châssis est déplaçable par rapport au socle, le panneau porte-patient est déplaçable en translation par rapport au châssis. Les déplacements du châssis peuvent permettre, d'une part, l'élévation afin d'accueillir en position basse les patients lorsque ces derniers se couchent sur le panneau porte-patient pour les élever ensuite de manière à ce que ces patients soient présentés d'une manière plus ergonomique au praticien qui est censé intervenir sur eux au moment de l'examen radiologique.

D'autre part, les châssis doivent aussi pouvoir être basculants, notamment dans les applications de myélographie. Alors que le patient est attaché sur le panneau, le châssis doit pouvoir prendre des positions verticales : les pieds du patient étant en bas et sa tête en haut, ou une position verticale inverse, dite aussi en Trendelenbourg, dans laquelle la tête du patient est orientée en bas. Les mouvements du panneau porte-patient par rapport au châssis sont normalement des mouvements de translation longitudinaux et latéraux, de manière à présenter certaines parties du corps du patient à l'irradiation X au moment de la prise du cliché, ou de l'étude radiologique.

Traditionnellement, on connaît deux familles de tables, compte tenu de la diversité des fonctionnalités à assurer. Une première famille concerne les tables utilisables en tomographie ainsi qu'en radiologie générale. Ces tables comportent essentiellement, du point de vue de l'invention, la fonction d'élévation ainsi que, d'une manière accessoire au problème présent, une possibilité d'orientation angulaire du faisceau de rayonnement X par rapport au patient. Cette angulation correspond aux besoins manifestés en tomographie. En pratique, le patient reste toujours, avec les tables de cette famille, couché horizontalement sur le panneau porte-patient. Il n'a par exemple pas besoin d'y être jamais attaché. Une telle solution est par exemple décrite dans le document EP-A-0 389 332.

L'autre famille de tables concerne les tables utilisables entre autres dans la fonction myélographie : essentiellement leur châssis est basculant dans les deux sens. En pratique, pour ce type de table basculante, trois philosophies existent. Dans un cas, avant de provoquer le basculement du châssis dans un sens ou dans un autre, on provoque son élévation de manière à ce que les extrémités de ce châssis ne viennent pas buter sur le sol au moment du basculement. Dans le deuxième cas, le mouvement de basculement est couplé à un mouvement de déplacement longitudinal du châssis, de manière à éviter le même problème. Bien entendu, dans les deux cas, des circuits électroniques de sécurité sont prévus pour empêcher toute action positive qui conduirait à une telle collision. Les cas 1 et 2 ont des basculements symétriques +90°/-90°. Une telle solution est par exemple décrite dans le document US-A-2 680 46.

Dans le troisième cas, le basculement du châssis est dissymétrique, c'est-à-dire 90° en verticale, et 15° à 20° de Trendelenbourg mais réalisé par un seul mécanisme. L'examen myélographique est exclu de l'utilisation mais ce type de châssis couvre la majorité des applications pour un prix de matériel avantageux. Il n'y a pas d'élévation pour ce type de table.

Le problème que doit résoudre l'industriel qui fabrique de telles tables est celui de la fabrication en série et à bas coût. On sait par exemple que, pour la fabrication de ces tables, les usines peuvent être agencées de la manière suivante. Des postes d'assemblages munis d'équipements spécialisés sont idéalement situés dans l'usine. Les tables, au fur et à mesure de leur achèvement, sont progressivement approchées de chacun de ces postes de travail. Si on fabrique trois types de tables, il faut prévoir une organisation triple en postes de travail et en équipements spécialisés. Ceci est d'une part lourd à administrer et par ailleurs cher : les équipements spécialisés étant eux-mêmes chers. Il apparaît donc nécessaire de réaliser des tables d'un genre unique qui puissent, en fonction de l'équipement dont elles seraient munies, avoir une fonctionnalité ou l'autre, ou même plusieurs.

En effet, dans les applications de radiologie classique et de tomographie, le châssis est normalement porté par un ascenseur de type vertical et motorisé, le panneau porte-patient étant motorisé en translations latérales et longitudinales sur le châssis. Par contre, pour les applications où le basculement est requis, et entre autres la myélographie, le châssis est solidaire d'un secteur circulaire, de forme sensiblement identique à celle d'un demi cercle. La périphérie de ce secteur circulaire est munie d'une crémaillère sur laquelle vient s'engrener une roue dentée fixée en bout d'arbre d'un moteur. Lorsque le moteur tourne, le secteur circulaire tourne sur lui-même en défilant devant l'arbre. Il entraîne alors le basculement du châssis verticalement dans un sens ou dans un autre. Cette solution à secteur circulaire, qui peut être remplacée par des systèmes à courroie crantée ou à chaîne, est peu compatible avec la réalisation d'un ascenseur, si ce n'est au prix d'une mécanique lourde et très complexe. Il en résulte que les familles de tables sont complètement différentes l'une de l'autre.

Une table de type basculant utilisable pour des examens en position non horizontale comme la myélographie est par exemple décrite dans la demande de brevet anglais GB-A-2 026 206. De même, on peut en voir d'autres exemplaires dans les demandes de brevet français n°s 2 224 963 et 2 542 604. Dans tous les cas on constate que l'adjonction d'un ascenseur au secteur permettant le basculement conduirait à une solution mécanique très complexe.

L'invention a pour objet de remédier aux inconvénients cités, notamment de résoudre les problèmes d'industrialisation, en proposant une structure de table qui puisse convenir d'une manière simple à la fabrication d'une table d'une famille ou de l'autre, ou même d'une table permettant les utilisations dans les deux familles. Le principe de l'invention consiste à utiliser, dans le mécanisme de liaison du socle au châssis une pièce intermédiaire appelée par la suite balancier. Ce balancier est mobile par rapport au socle, le châssis étant mobile par rapport au balancier. En pratique le châssis n'a aucune liaison directe au socle, si ce n'est par l'intermédiaire du balancier. En simplifiant l'explication, on peut dire que si on utilise un seul moteur, on pourra avoir une utilisation selon le troisième mode de travail. Par contre, si on munit la table de deux moteurs, elle pourra évidemment travailler selon le premier ou le deuxième mode de travail mais aussi selon le troisième.

L'invention a donc pour objet une table d'examen médical comportant un socle et un châssis pour porter un panneau porte-patient, caractérisée en ce qu'elle comporte un balancier intermédiaire assurant la liaison mécanique du socle au châssis, deux axes de rotation, et des moyens moteurs pour provoquer avec des rotations autour de ces axes, le déplacement du balancier par rapport au socle et du châssis par rapport au balancier.

L'invention sera mieux comprise à la lecture de la description qui suit et à l'examen des figures qui l'accompagnent. Celles-ci ne sont données qu'à titre indicatif et nullement limitatif de l'invention. Les figures montrent :

- figure 1 : une vue de face d'une table d'examen selon l'invention ;
- figure 2 : une vue de dessus de la table de la figure 1 ;
- figure 3a et 3b et figure 4 : des vues de face de positions en inclinaison de la table de la figure 2 ;
- figure 5 : une représentation schématique du domaine de l'espace accessible avec la table de l'invention ;
- figure 6 : un dispositif de commande automatique des différents moteurs de la table pour assurer un déplacement particulier.

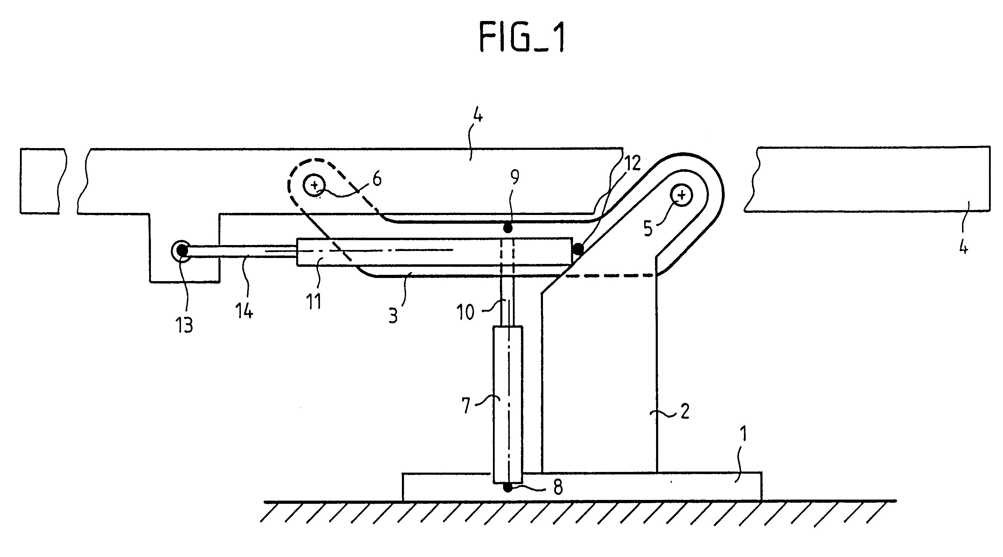

La figure 1 montre une table d'examen médical selon l'invention. Cette table comporte un socle composé d'un piédestal 1 et d'un mât 2, appliqué sur le sol. Pour diverses raisons d'asymétrie de basculement, le socle peut être déporté à droite ou à gauche de la table. Sur le mât 2 est monté en rotation un balancier 3. Sur le balancier 3 est monté en rotation un châssis 4 de la table. La liaison du mât 2 au balancier 3 est réalisée au moyen d'un arbre 5 permettant la rotation du balancier 3. La liaison du balancier 3 au châssis 4 est réalisée au moyen d'un arbre 6 qui permet la rotation du châssis 4 par rapport au balancier. Le panneau porte-patient n'est pas représenté. Il est normalement placé au dessus du châssis.

Pour conduire ces rotations, on dispose dans l'invention, de un ou deux moteurs. Les moteurs sont ici des vérins, de préférence des vérins mécaniques ou hydrauliques différentiels double effet, à clapets anti-retour (pour la sécurité) si hydrauliques, dont seule la fonction a été représentée, et non pas les circuits d'alimentation en fluide. Un premier vérin 7 prend appui d'une part sur le socle 1 par un pivot 8 et, d'autre part, sur le balancier 3 par un pivot 9. Pour une position donnée d'extension de la tige 10 du vérin 7, le balancier 3 présente par rapport au mât 2 une orientation donnée. La rotation du châssis 4 autour du balancier 3 est conduite par un vérin 11 fixé par un premier pivot 12 au balancier, et fixé par un deuxième pivot à une extrémité 13 au châssis 4. Là également pour une extension donnée de la tige 14 du vérin 11, le châssis 4 prend une orientation donnée par rapport au balancier 3. Les pivots 8, 9, 12 et 13 sont de préférence réalisés avec des petits arbres dont les axes de rotation sont, comme les axes de rotation des arbres 5 et 6, perpendiculaires au plan de la figure 1.

Les différents arrachés et lignes en tirets de la figure 1 permettent de comprendre que le châssis est tenu uniquement par le pivot 13 et par l'arbre 6 et que le balancier est tenu uniquement par l'arbre 5 et le pivot 9. En conséquence, on en déduit que les extrémités du châssis 4 sont en porte-à-faux par rapport à ses fixation aux arbres 6 et 13.

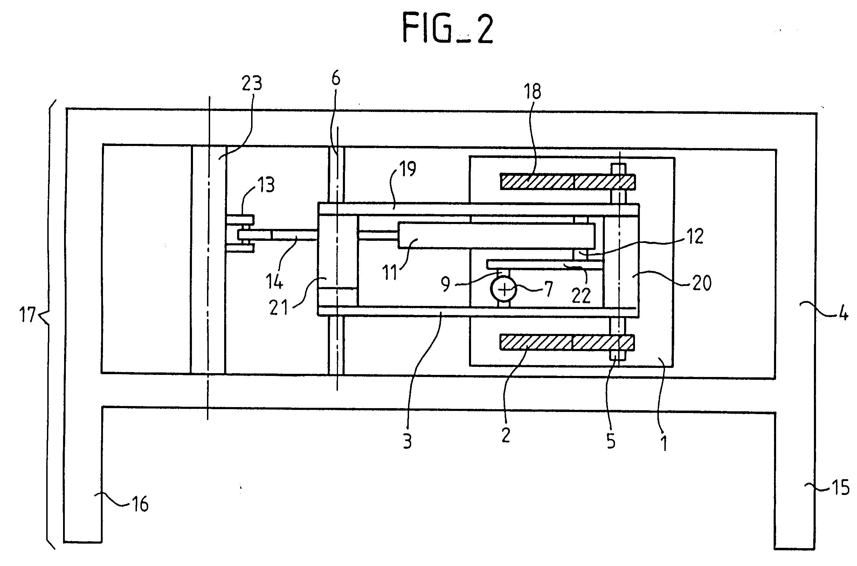

La figure 2 montre les mêmes éléments vus de dessus. Le châssis comporte un cadre rectangulaire muni de deux ailes 15 et 16 en porte-à-faux, du côté bas de la figure. En pratique, le praticien se place du côté de ces ailes 15 et 16, et ses pieds ne viennent ainsi pas buter sur le piédestal 1. Normalement, le sommet du mât 2 n'est pas prévu pour passer complètement au travers du châssis 4, de sorte que le panneau porte-patient et/ou les autres éléments constituant la table peuvent être posés sur la totalité de la surface du châssis. De plus, le panneau porte-patient n'occupe pas toute cette surface mais, en étant moins large que la largeur 17 de ce châssis, il peut y être déplacé latéralement d'une manière classique.

La figure 2 permet de voir que le mât 2 est en fait constitué principalement de deux mâts parallèles plats et en potence 2 et 18, pièces maîtresses du socle. Dans un exemple préféré de réalisation, les mâts 2 et 18 sont fixés d'une manière mécanosoudée sur le piédestal 1. A leurs sommets, il maintiennent par des paliers l'arbre 5. Le balancier 3 est lui également muni, dans la réalisation préférée montrée, essentiellement de deux longerons métalliques plats respectivement 3 et 19. Comme représenté sur la figure 1, chacun de ces longerons peut avoir sensiblement une forme en cuvette. Au sommet des bords de chacune de ces cuvettes sont montés des paliers dans lesquels s'engagent les arbres 5 et 6. Les bras 3 et 19 du balancier sont fixés l'un à l'autre par mécanosoudure, avec deux entretoises 20 et 21 creuses, de part et d'autre de leurs extrémités.

Une console plate 22 est également fixée à l'entretoise 20, au travers de laquelle passe l'arbre 5. Elle y est aussi soudée. Cette console 22 permet la fixation des deux pivots 9 et 12. L'arbre du pivot 9 est fixé entre la console 22 et le bras 3 du balancier. L'arbre du pivot 12 est fixé entre la console 22 et le bras 19 du balancier. Les vérins 7 et 11 sont respectivement articulés sur chacun de ces pivots avec des paliers à leurs extrémités. A son extrémité gauche sur le dessin, le châssis 4 comporte une entretoise 23 à laquelle est fixé l'arbre du pivot 13 sur lequel vient s'articuler l'extrémité du vérin 11. Dans un exemple, la longueur du châssis est de l'ordre de 900 mm, la longueur du balancier entre les arbres 5 et 6 et de l'ordre de 835 mm.

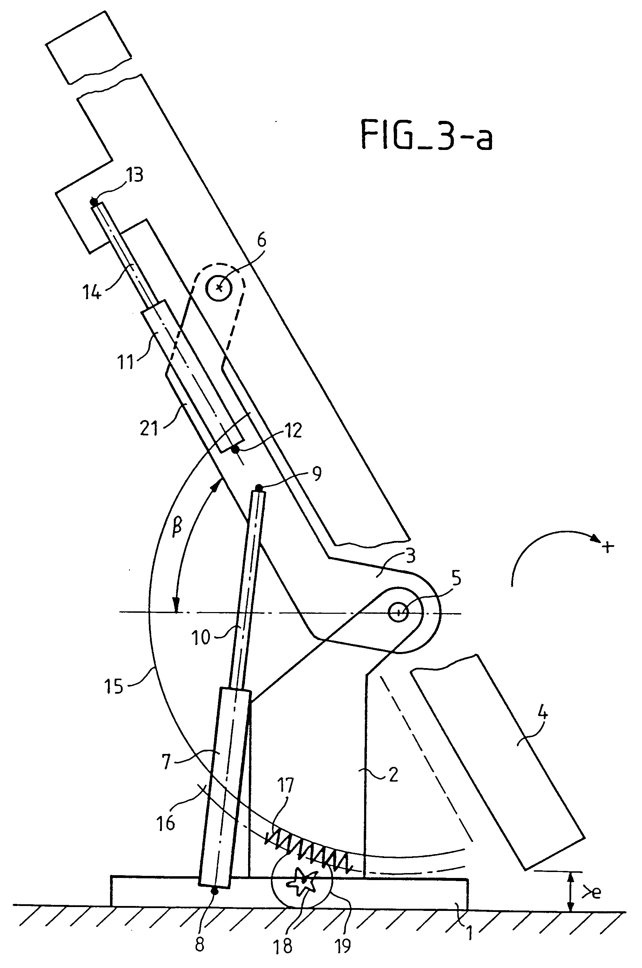

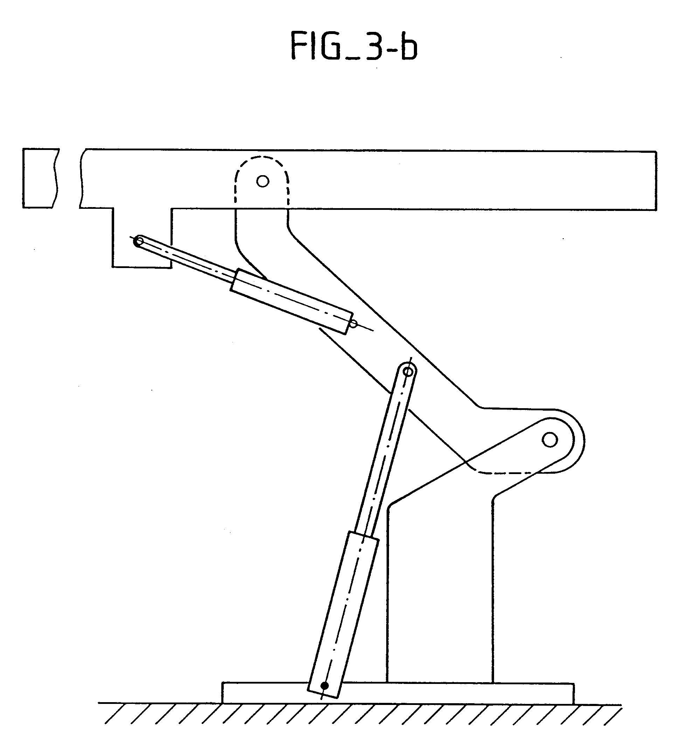

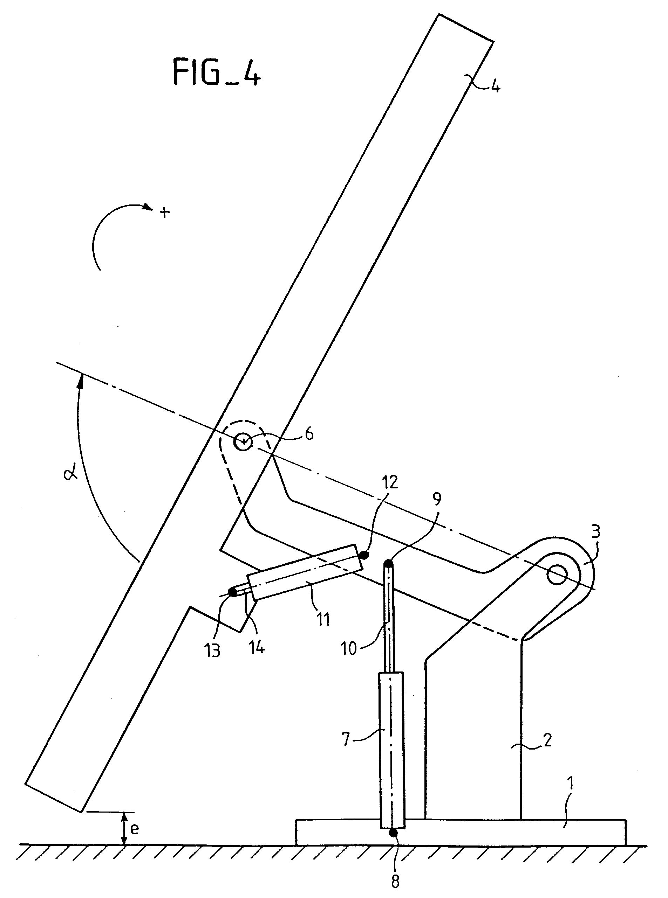

Les figures 3a,3b et 4 montrent les diverses positions prises par la table de l'invention lorsque l'un ou l'autre ou bien l'un et l'autre des vérins 7 et II sont étendus. La figure 3 montre l'angle d'inclinaison $\beta$ que prend le balancier 3 par rapport à l'horizontale, ou plutôt par rapport au socle 1. La figure 4 quant à elle montre l'orientation $\alpha$ que prend le châssis 4 par rapport au balancier 3.

Les pivots de fixation 12 et 9 ont été déplacés, sur les figures 3 et 4, par rapport aux positions qu'ils occupaient sur les figures 1 et 2, d'une part pour simplifier l'explication et d'autre part pour montrer aussi que ces pivots peuvent avoir, sur le balancier, d'autres positions que les positions préférées indiquées dans les figures 1 et 2. Dans les représentations des figures 3 et 4, la cinématique des mouvements est modifiée par la position des pivots. Mais les principes de l'invention restent inchangés. On pourra adapter cette position des pivots à une cinématique voulue. Ainsi, les vérins pourront avoir par exemple des fonctionnements inverses ; c'est-à-dire qu'un vérin agissant par exemple en poussée pour provoquer un basculement défini, peut par un choix différent de la position de ses pivots, agir en traction pour provoquer le même basculement précité. De même, au lieu d'être tous les deux articulés sur le balancier, les vérins peuvent être tous les deux articulés sur le socle ou sur le châssis. Dans ces cas, les vérins sont respectivement fixés par leur arbres au socle et au balancier et au socle et au châssis, ou bien au socle et au châssis et au balancier et au châssis.

Sur la figure 3a, et en supposant que le patient ait placé ses pieds du côté droit du châssis 4, il est relevé en position verticale en agissant uniquement sur l'extension du vérin 7 et en maintenant par ailleurs le vérin 11 dans sa position initiale. Par contre, figure 4, dans la position dite en Trendelembourg, on a rentré la tige 14 du vérin 11 dans le corps de ce vérin et la tête du patient se retrouve en bas.

On constate, sur la figure 4, que, d'une manière préférée, quand on agit sur la tige du vérin 14 pour qu'elle se retire à l'intérieur du corps du vérin, on a au préalable fait sortir un peu la tige 10 du vérin 7. Ceci permet d'obtenir d'une part un écart de sécurité e qui évite au châssis 4 de venir buter sur le sol et d'autre part, de maintenir le basculement de la table sous une hauteur de plafond de salle minimum. Ceci a par ailleurs un autre avantage : le mécanisme décrit permet l'élévation. En effet, d'une manière préférée, la hauteur des mâts 2 et la position du châssis 4, comme montrées sur la figure 1, sont prévues de telle façon que le panneau porte-patient soit assez bas, proche du sol. Dans ces conditions, le patient peut facilement s'y asseoir pour s'y étendre. En pratique, cette position amène le panneau porte-patient à être à une hauteur inférieure à 70 cm au dessus du sol. Par contre, soit pour l'utilisation en Trendelembourg, soit tout simplement pour faciliter l'intervention du praticien, on préfère élever le châssis par une action combinée d'extension de la tige 10 du vérin 7 et de rétraction de la tige 14 du vérin 11. On conçoit par exemple, figure 3, que si, dans la position indiquée, on provoque la rétraction de la tige 14 dans le corps du vérin 11, le châssis va se retrouver parallèlement au sol à une altitude élevée, on a ainsi défini pour l'appareil une "grande hauteur" qui place en pratique le panneau porte-patient à environ 125 cm au dessus du sol. Cf. figure 3 b.

La table de l'invention permet de résoudre efficacement les problèmes d'industrialisation évoqués auparavant ci-dessus. En effet, une même structure de table sera retenue pour fabriquer des tables qui sont susceptibles de s'élever seulement ou de basculer seulement ou pour fabriquer des tables susceptibles de s'élever et de basculer quelle que soit l'amplitude des mouvements. En effet, pour fabriquer des tables qui sont utilisables avec une élévation seule, donc sans la possibilité de basculement, l'action des moteurs sera couplée. En pratique, on s'arrangera pour que l'angle $\alpha$ soit égal à l'angle $\beta$. Cette égalité est donc une relation linéaire. On sait réaliser mécaniquement et simplement, des rotations liées entre elles par une relation linéaire. Par exemple, il suffit de remplacer le vérin 7 par un moteur rotatif entraînant, au besoin avec une chaîne, la rotation du balancier autour du socle. Par un système d'engrenage, avec un coefficient multiplicateur égal à 1, il suffit, également au besoin avec une autre chaîne, de provoquer une rotation identique du châssis autour du balancier. Dans ce cas, on a un seul moteur, et on provoque par action dans un sens ou dans un autre sur ce moteur, l'élévation ou l'abaissement de la table.

Il en résulte que, pour une table, susceptible d'élévation, la complexité du mécanisme est faible. En effet, il y a seulement la présence du balancier intermédiaire avec un arbre de rotation, l'arbre 6. Par contre, on peut facilement passer à l'utilisation en mode basculant, en munissant la table d'un deuxième moteur indépendant, ou partiellement couplé au premier, et en permettant les basculements évoqués dans les figures 3a, 3b et 4. On notera que cette technique permet la modification des tables chez le client lui-même, dans la mesure où le client aurait acheté au début une table simple et où il désirerait par la suite perfectionner son équipement.

Par opposition aux solutions de l'état de la technique, l'invention présente donc un deuxième arbre de rotation, l'arbre 6, qui permet de faire tourner le châssis 4, par rapport au balancier intermédiaire 3 ou 15. Bien entendu, d'autres formes de réalisation sont utilisables. Ce qui est essentiel est la présence, dans le dispositif de l'invention, des deux centres de rotation ici les arbres 5 et 6.

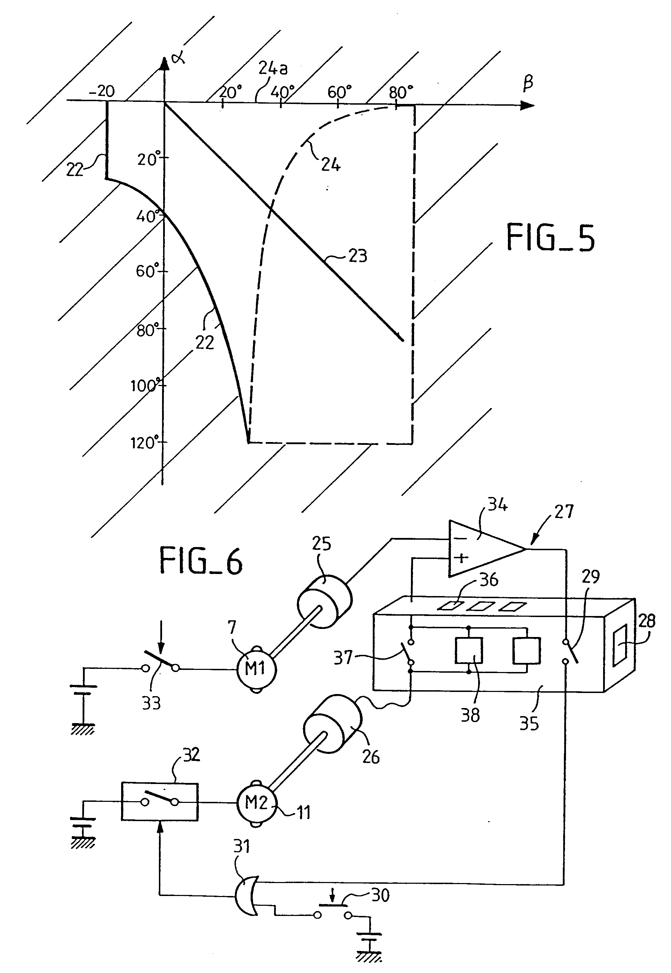

La figure 5 montre le domaine exploitable de l'orientation du châssis par rapport au balancier et du balancier par rapport au socle. Les angles sont mesurés avec leur valeur positive dans le sens trigonométrique. Dans l'exemple montré, l'angle $\beta$ peut être légèrement négatif, l'angle $\alpha$ reste toujours négatif. La courbe 22, tout autour, montre la limite du domaine interdit de l'angle $\alpha$ pour des valeurs données de l'angle $\beta$. Cette courbe peut être chargée dans une mémoire indiquant la relation entre $\alpha$ et $\beta$. La courbe 23 montre l'opération d'élévation de la table : $\beta = \alpha$. La courbe 24 montre une cinématique possible que l'on utilisera pour passer d'un angle $\beta = 90°$ (avec $\alpha = 0°$) à un angle $\alpha$ 120° (avec un angle $\beta$ 30°, ce qui conduit à une orientation verticale inverse : 120° - 30° = 90°). La courbe 24 bis montre l'opération de basculement vers la verticale. Les parties hachurées du diagramme montrent les positions inutilisables.

A l'aide de la figure 6 on va montrer comment on peut, de manière schématique, faire fonctionner les différents moteurs. Cette figure montre les moteurs 7 et 11 sous une forme symbolique M1 et M2. L'arbre de chacun de ces moteurs est relié, respectivement, à un synchrodétecteur 25 et 26. Ces synchrodétecteurs sont par exemples montés sur les arbres 5 et 6 respectivement. Ces synchrodétecteurs délivrent des signaux électriques utilisables dans un circuit de commande et de régulation 27. Ce circuit de commande et de régularisation 27 comporte premièrement une commande 28 qui autorise l'indépendance des moteurs.

En pratique, et d'une manière schématique, lorsqu'un interrupteur 29 lié à 28 est ouvert, la mise en service du moteur M2 est liée uniquement à une action sur un interrupteur 30 qui, au travers d'une porte logique OU 31 agit sur un relais 32. Le moteur M1 lui est mis en service par action positive sur un interrupteur 33.

Par contre, lorsque le mouvement des moteurs doit être couplé, par exemple pour provoquer une élévation (courbe 23) ou pour provoquer un basculement inverse (courbe 24), l'interrupteur 29 est fermé. Dans ce cas, les signaux des synchrodétecteurs 25 et 26 sont envoyés à un comparateur 34. L'un des signaux, celui qui provient du moteur M2, est transmis par l'intermédiaire d'un circuit de régulation 35. Ce circuit de régulation 35 possède plusieurs modes de fonctionnement. Chacun de ces modes est mis en service par une action sur des boutons de commande tels que 36.

Dans le cas où on provoque l'élévation, un de ces boutons 36 a pour fonction de fermer (schématiquement) un interrupteur 37 qui permet la comparaison directe, dans le comparateur 34, des signaux émis par les synchrodétecteurs 25 et 26. Le comparateur 34 délivre donc un signal d'erreur qui est alors transmis, par l'intermédiaire de la porte 31 au relais 32. Ceci a pour effet de faire tourner le moteur M2 dans un sens tel que l'égalité des angles $\beta$ et $\alpha$ soit conservée.

Pour respecter une courbe 24 de basculement de -90° à +90° on utilisera au contraire des mémoires telles que 38. Ces mémoires reçoivent, en entrée d'adresse, la mesure de l'angle a effectuée par le synchrodétecteur 26 et délivrent en sortie une valeur de l'angle β qui leur correspond sur la courbe 24. Une numérisation de ces fonctions est mise en oeuvre en utilisant des convertisseurs analogique-numérique en entrée des mémoires et numérique-analogique en sortie. Le comparateur 34 compare alors la mesure de l'angle β réel, à celle que β devrait avoir si l'équipement se trouvait sur la courbe 24. Le comparateur délivre alors un signal d'erreur propre à agir sur le moteur M2 de façon que le passage par cette courbe soit respecté. Etant donné qu'il peut y avoir plusieurs modes de ralliement possibles : passage de l'élévation au basculement ou bien passage du basculement à la position basse pour que le patient puisse descendre de la table, il peut y avoir lieu de prévoir plusieurs tables mémorisées, telles que 38, mises en service chacune par des boutons tels que 36.

## Revendications

1. Table d'examen médical comportant un socle (1,2) et un châssis (4) pour porter un panneau porte-patient, un balancier (3) intermédiaire assurant la liaison mécanique du socle au châssis par deux axes (5,6) de rotation montés aux extrémités du balancier, et des moyens moteurs (7,11) pour provoquer avec des rotations autour de ces axes le déplacement du balancier par rapport au socle et du châssis par rapport au balancier, caractérisée en ce que les moyens moteurs sont constitués de deux vérins de déplacement, un premier vérin double effet (7) pour déplacer le balancier par rapport au socle et un deuxième vérin (11) double effet pour déplacer le châssis par rapport au balancier, afin de permettre des déplacement en élévation et des déplacement en basculement de la table.

2. Table selon la revendication 1, caractérisée en ce qu'elle comporte des moyens (27) pour coupler les deux vérins et provoquer ainsi un déplacement particulier du châssis par rapport au socle.

3. Table selon la revendication 2, caractérisée en ce qu'elle comporte des moyens (37) pour provoquer une élévation (23) du châssis parallèlement à lui même.

4. Table selon l'une quelconque des revendications 1 à 3, caractérisée en ce que les deux vérins (7,11) de déplacement sont fixés respectivement au socle (1) et au balancier (3) et au balancier (3) et au châssis (4).

5. Table selon l'une quelconque des revendications 1 à 3, caractérisée en ce que les deux vérins (7,11) de déplacement sont fixés respectivement au socle (1) et au balancier (3), et au socle (1) et au châssis (4).

6. Table selon l'une quelconque des revendications 1 à 3, caractérisée en ce que les deux vérins (7,11) de déplacement sont fixés respectivement au socle (1) et au châssis (4), et au balancier (3) et au châssis (4).

7. Table selon l'une quelconque des revendications 4 à 6, caractérisée en ce que les vérins sont des vérins hydrauliques ou mécaniques.

8. Table selon la revendication 7, caractérisée en ce que les vérins hydrauliques comportent des clapets anti-retour.

9. Table selon l'une quelconque des revendications 1 à 8, caractérisée en ce que le panneau porte-patient est disposé sur le châssis en porte-à-faux par rapport à l'aplomb de l'endroit où est placé le balancier.

## Claims

1. Medical examination table comprising a stand (1, 2) and a frame (4) serving to bear a patient-supporting panel, an intermediate rocker (3) providing the mechanical connection of the stand to the frame by two rotation shafts (5, 6) mounted at the ends of the rocker, and motor means (7, 11) for moving the rocker in relation to the stand and moving the frame in relation to the rocker by rotations about the said shafts, characterised by the fact that the motor means consist of two displacement jacks, a first double-action jack (7) for displacing the rocker in relation to the stand and a second double-action jack (11) for displacing the frame in relation to the rocker, in order to enable the table to perform elevation movements and tilting movements.

2. Table according to claim 1, characterised by the fact that it comprises means (27) for coupling the two jacks and thus effecting a particular displacement of the frame in relation to the base.

3. Table according to claim 2, characterised by the fact that it comprises means (37) for causing the frame to perform a parallel elevation movement.

4. Table according to any one of claims 1 to 3, characterised by the fact that the two displacement jacks (7, 11) are affixed to the base (1) and the rocker (3) and the frame (4) respectively.

5. Table according to any one of claims 1 to 3, characterised by the fact that the two displacement jacks (7, 11) are affixed to the base (1) and the rocker (3) and to the base (1) and the frame (4) respectively.

6. Table according to any one of claims 1 to 3, characterised by the fact that the two displacement jacks (7, 11) are affixed to the base (1) and the frame (4) and to the rocker (3) and the frame (4) respectively.

7. Table according to any one of claims 4 to 6, characterised by the fact that the jacks are hydraulic or mechanical jacks.

8. Table according to claim 7, characterised by the fact that the hydraulic jacks comprise non-return valves.

9. Table according to any one of claims 1 to 8, characterised by the fact that the patient-supporting panel is positioned on the frame with an overhang in relation to the perpendicular of the place where the rocker is provided.

**Patentansprüche**

1. Medizinischer Untersuchungstisch mit einem Sockel (1, 2) und einem Gestell (4) zur Aufnahme einer den Patienten tragenden Platte, einer dazwischen angeordneten Wippe (3) zur mechanischen Verbindung des Sockels mit dem Gestell über zwei Drehachsen (5, 6), die an den Enden der Wippe befestigt sind und einer Motoranordnung (7, 11), um eine Bewegung der Wippe bezüglich des Sockels und des Gestells bezüglich der Wippe um die Drehachsen herum zu bewirken, dadurch gekennzeichnet, daß die Motoranordnung aus zwei die Bewegungen bewirkenden Druckzylindern besteht, und zwar aus einem ersten doppelt wirkenden Druckzylinder (7) zur Bewegung der Wippe bezüglich des Sockels und aus einem zweiten doppelt wirkenden Druckzylinder (11) zur Bewegung des Gestells bezüglich der Wippe, um so sowohl Hebebewegungen als auch Schwenkbewegungen des Tisches zu ermöglichen.

2. Tisch nach Anspruch 1, dadurch gekennzeichnet, daß er eine Anordnung (27) aufweist, um die beiden Druckzylinder miteinander zu koppeln und derart eine besondere Bewegung des Gestells bezüglich des Sockels zu bewirken.

3. Tisch nach Anspruch 2, dadurch gekennzeichnet, daß er eine Anordnung (37) aufweist, um einen Hub (23) des Gestells parallel zu sich selbst zu bewirken.

4. Tisch nach einem der Ansprüche 1 bis 3, dadurch gekennzeichnet, daß die beiden die Bewegungen bewirkenden Druckzylinder (7, 11) am Sockel (1) und an der Wippe (3) bzw. an der Wippe (3) und am Gestell (4) befestigt sind.

5. Tisch nach einem der Ansprüche 1 bis 3, dadurch gekennzeichnet, daß die beiden die Bewegungen bewirkenden Druckzylinder (7, 11) am Sockel (1) und an der Wippe (3) bzw. am Sockel (1) und am Gestell (4) befestigt sind.

6. Tisch nach einem der Ansprüche 1 bis 3, dadurch gekennzeichnet, daß die beiden die Bewegungen bewirkenden Druckzylinder (7, 11) am Sockel (1) und am Gestell (4) bzw. an der Wippe (3) und am Gestell (4) befestigt sind.

7. Tisch nach einem der Ansprüche 4 bis 6, dadurch gekennzeichnet, daß die Druckzylinder hydraulische oder mechanische Druckzylinder sind.

8. Tisch nach Anspruch 7, dadurch gekennzeichnet, daß die hydraulischen Druckzylinder Rückschlagventile aufweisen.

9. Tisch nach einem der Ansprüche 1 bis 8, dadurch gekennzeichnet, daß die den Patienten tragende Platte derart angeordnet ist, daß sie aus dem Gestell senkrecht zu der Stelle, an der die Wippe angeordnet ist, herausragt.

FIG_1

# FIG_2

EP 0 511 049 B1

FIG_3-a

# FIG_3-b

FIG_4

FIG_5

FIG_6